# EUROPEAN PATENT SPECIFICATION

(11) **EP 3 700 510 B1**
(45) Date of publication and mention of the grant of the patent: **06.10.2021**
(21) Application number: 18792925.2
(22) Date of filing: 23.10.2018
(51) Int. Cl.: A61K 9/48, A61K 31/506, A61K 31/519, A61K 31/53

(54) **LIQUID FILLED FORMULATIONS OF PDE5 INHIBITORS**
FLÜSSIGKEITSGEFÜLLTE FORMULIERUNGEN VON PDE5-INHIBITOREN
FORMULATIONS REMPLIES LIQUIDES D'INHIBITEURS DE PDE5

(30) Priority: 26.10.2017 US 201762577605 P
(43) Date of publication of application: 02.09.2020
(73) Proprietor: Capsugel Belgium NV, 2880 Bornem (BE)
(72) Inventor: SCAMMELLS, Peter John, Melbourne Victoria 3104 (AU); PORTER, Christopher, John, Hamilton, South Melbourne Victoria 3205 (AU); BENAMEUR, Hassan, 69003 Lyon (FR); WILLIAMS, Hywel, David, Melbourne Victoria 3046 (AU); FORD, Leigh, North Melbourne Victoria 3052 (AU)
(74) Representative: De Clercq & Partners
(86) International application number: PCT/EP2018/078943
(87) International publication number: WO 2019/081451

(56) References cited:
- WO-A1-2017/168174
- WO-A2-2011/030351
- Althaf ET AL: "FORMULATION AND EVALUATION OF ORAL FAST DISSOLVING TABLETS OF SILDENAFIL CITRATE", International Journal of Pharmacy and Pharmaceutical Sciences, 13 December 2010 (2010-12-13), pages 112-121, XP055199110, Retrieved from the Internet: URL:http://www.ijppsjournal.com/Vol3Suppl2 /1127.pdf [retrieved on 2015-06-30]

## Description

The present application relates to liquid filled formulations of PDE5 inhibitors.

### BACKGROUND

Phosphodiesterase type 5 (PDE5) inhibitors are used for the treatment of erectile dysfunction (ED) as well as other clinical indications such as pulmonary hypertension in adults and in children. Sildenafil is a specific example of a PDE5 inhibitor compound and is currently formulated as an oral tablet dosage form e.g., Viagra® for treatment of ED. Other marketed products for the ED indication include vardenafil (Levitra®) and avanafil (Stendra®), also as tablet dosage forms. These actives are basic, having at least one basic pKa above about 5 and therefore may be formulated starting from the crystalline free base or a salt. There are however numerous counterfeit products available, which creates considerable challenges to patient safety, lost revenue, litigation, brand erosion, efficacy question marks etc.

In addition to tablet products, an orally disintegrating tablet and a chewable softgel sildenafil product are marketed in non-US markets. These products are designed to achieve a more rapid onset of action when compared to the oral tablet, however they do suffer from poor palatability.

Sildenafil is also licensed in the US and other countries for pulmonary hypertension treatment in adults (Revatio® tables) and is currently under clinical investigation in children for the same indication. In this pediatric application, the development of a palatable oral formulation is crucial to patient compliance.
WO 2017/168174A1 discloses fatty acid salts of sildenafil, such as sildenafil dodecanoate admixed with excipients in multicomponent pharmaceutical compositions. A first component is adapted to deliver sildenafil rapidly to promote fast onset of action, while a second component is adapted to deliver the sildenafil from dose to dose.
WO 2011/030351A2 discloses taste-masked pharmaceutical compositions comprising PDE5 inhibitors. Oral compositions are disclosed that comprise at least one PDE5 inhibitor, at least one taste-masking agent and at least one pharmaceutically acceptable excipient.

There is therefore a need for new and innovative oral formulations of PDE5 inhibitors.

### SUMMARY

The scope of the invention is defined by the claims appended hereto. Any subject-matter falling outside the scope of the claims is provided for information purposes only.

Disclosed are formulations of PDE5 inhibitors. In certain examples, the disclosed composition comprises a capsule and a fill formulation, wherein the fill formulation comprises an active ingredient comprising, consisting of or consisting essentially ofsildenafil, vardenafil, avanafil, udenafil, mirodenafil, lodenafil or any combination thereof, a lipophilic counterion to the active ingredient, the lipophilic counterion being present in an amount of at least 90 mol% of the active ingredient so as to be capable of forming a lipophilic salt of the active ingredient, and a lipid vehicle that is liquid or predominantly liquid at 25°C, wherein the active ingredient is completely or substantially completely dissolved in the lipid vehicle in an amount of at least 1.0%wt at 25°C (expressed as free base equivalents) of the fill formulation. In particular examples the active ingredient is sildenafil.

In any of the examples the composition may comprise the active ingredient present in the composition in an amount of at least 2.5wt% (expressed as free base equivalents). In any of the examples the lipid vehicle may comprise, consist essentially of or consist of a surfactant or a mixture of surfactants, optionally a cosurfactant, and optionally an oil. In any example having a surfactant(s), the surfactant or surfactants may be polyoxyethylene sorbitan fatty acid esters, a mixture of (i) polyoxyethylene mono- and di-esters of C8-C22 fatty acids and (ii) glyceryl mono-, di-, and tri-esters of C8-C22 fatty acids, polyoxyethylene castor oils and derivatives, polyoxyethylene fatty acid esters, Vitamin E TPGS or one or more derivatives thereof, polyoxyethylene-polyoxypropylene copolymers, or any combination thereof. In certain examples the surfactant is a polyoxyethylene sorbitan fatty acid ester. The polyoxyethylene sorbitan fatty acid ester may be polysorbate 20, polysorbate 40, polysorbate 60, polysorbate 80, polysorbate 85, or any combination thereof. In some examples the surfactant is a mixture of (i) polyoxyethylene mono- and di-esters of C8-C22 fatty acids and (ii) glyceryl mono-, di-, and tri-esters of C8-C22 fatty acids selected from the group consisting of caprylocaproyl macrogol-8 glycerides, oleoyl macrogol-6 glycerides or linoleoyl macrogol-6 glycerides, lauroyl macrogol-32 glycerides, stearoyl macrogol-32 glycerides and macrogol stearate. In other examples the surfactant is a polyoxyethylene castor oil. In certain examples polyoxyethylene castor oil is polyoxyl 35 castor oil, polyoxyl 40 hydrogenated castor oil, polyoxyl 60 hydrogenated castor oil orany combination thereof. In other examples the surfactant is a polyoxyethylene fatty acid ester. In some examples the polyoxyethylene fatty acid esters is polyoxyl 40 stearate, polyoxyl 40 oleate, polyoxyl 8 stearate, polyoxyl 15 hydroxystearate orany combination thereof.

In certain examples the surfactant is Vitamin E TPGS and/or a derivative thereof. In other examples the surfactant is a polyoxyethylene-polyoxypropylene copolymer, such as poloxamer 124, poloxamer 188, poloxamer 407 or any combination thereof.

Some examples of the disclosed formulations include a cosurfactant that comprises, consists essentially of or consists of propylene glycol mono- and di-esters of C8-C22 fatty acids, sorbitan fatty acid esters or a mixture thereof. In other examples the cosurfactant is a propylene glycol mono- or di-ester of C8-C22 fatty acids comprising, consisting essentially thereof or consisting of propylene glycol monocaprylate, propylene glycol dicaprolate/dicaprate, propylene glycol monolaurate orany combination thereof. In yet other examples the cosurfactant is a sorbitan fatty acid esters comprising sorbitan monolaurate, sorbitan monopalmitate, sorbitan monooleate, sorbitan trioleate orany combination thereof.

In any of the examples the oil may comprise, consist essentially of or consist of a C8-C18 fatty acid ester of glycerol. Alternatively, the oil is a C8 to C18 triglyceride. In yet other examples the oil is a mixture of C8 to C18 mono-, di- and/or triglycerides. In some examples the oil is almond oil, babassu oil, blackcurrant seed oil, borage oil, canola oil, castor oil, coconut oil, cod liver oil, corn oil, cottonseed oil, evening primrose oil, fish oil, grape seed oil, mustard seed oil, olive oil, palm kernel oil, palm oil, peanut oil, rapeseed oil, safflower oil, sesame oil, shark liver oil, soybean oil, sunflower oil, walnut oil, wheat germ oil, avocado oil, bran oil, hydrogenated castor oil, hydrogenated coconut oil, hydrogenated cottonseed oil, hydrogenated palm oil, hydrogenated soybean oil, partially hydrogenated soybean oil, hydrogenated vegetable oil, caprylic/capric glycerides, fractionated triglycerides, glyceryl tricaprate, glyceryl tricaproate, glyceryl tricaprylate, glyceryl tricaprylate/caprate, glyceryl tricaprylate/caprate, glyceryl tricaprylate/caprate/laurate, glyceryl tricaprylate/caprate/linoleate, glyceryl tricaprylate/caprate/stearate, glyceryl trilaurate or any mixture thereof. In any of the examples the oil may comprise, consist essentially of or consist of glyceryl laurate, glyceryl linoleate, glyceryl oleate, glyceryl stearate, glyceryl caprylate, glyceryl caprate or any mixture thereof.

In any of the examples the lipid vehicle may comprise, consist essentially of or consist of a medium-chain triglyceride, propylene glycol monocaprylate and polyoxyl 35 castor oil. In certain examples the lipid vehicle comprises a propylene glycol monocaprylate, PEG-8 caprylic/capric glycerides and polyoxyl 35 castor oil.

In any of the examples the lipophilic salt of the active ingredient may have a solubility in the lipid vehicle that is at least 5-fold greater than the solubility of the commercial (e.g., citrate in the case of sildenafil or hydrochloride in the case of vardenafil) salt form of the active ingredient in the lipid vehicle. In certain examples the active ingredient is sildenafil and the lipophilic salt of the active ingredient has a solubility in the lipid vehicle that is at least 10-fold greater than the solubility of the citrate salt form of the active ingredient in the lipid vehicle. In other examples the active ingredient is sildenafil and the lipophilic salt of the active ingredient has a solubility in the lipid vehicle that is at least 25-fold greater than the solubility of the citrate salt form of the active ingredient in the lipid vehicle. In some examples the active ingredient is sildenafil and the lipophilic salt of the active ingredient has a solubility in the lipid vehicle that is at least 50-fold greater than the solubility of the citrate salt form of the active ingredient in the lipid vehicle.

In any of the examples the counterion may comprise, consist essentially of or consist of an organic molecule containing carbon atoms. In certain examples the counterion has at least one acidic group with a pKa value of less than 7, and may include at least one acidic group with a pKa value of less than 4.

In any of the examples the counterion may have a LopP or cLogP that is greater than 0, and more preferably is greater than 2.

In any of the examples the counterion may have a molecular weight such that the counterion:active ingredient molar mass ratio in the salt is less than 2.5, or the counterion active ingredient molar mass ratio in the salt is less than 1.5. In certain examples the lipophilic counterion comprises, consists essentially of or consists of carboxylic acids (RC(O)O-), phosphates (ROP(O)O2-), phosphonates (RP(O)O2-), sulfonates (RS(O)2O-), sulfates (ROS(O)2O-), tetrazolyls (R-tetrazolate) and/or bis(sulfonyl)imides (RSO2-N--SO2R) where R may be any suitable group, such as an optionally substituted hydrocarbon group containing between 2 and 24 carbon atoms, and where this hydrocarbon R group is a saturated straight chained or branched hydrocarbon or a saturated cyclic hydrocarbon or an unsaturated cyclic hydrocarbon, and where the R group is unsubstituted or may be substituted by 1, 2, 3, 4, 5, or 6 or more same or different optional substituents. In other examples the lipophilic counterion isdecylsulfate, lauryl sulfate, 7-ethyl-2-methyl-4-undecylsulfate, dioctylsulfosuccinate (docusate), oleate, stearate, palmitate, laurate (dodecanoate), caprate (decanoate), caprylate (octanoate), butyl octyl sulfate or any combination thereof. In yet other examples the lipophilic counterion is lauryl sulfate, dioctylsulfosuccinate (docusate), decyl sulfate or any combination thereof.

In any of the examples the active ingredient may be present in an amount of at least 2.5wt% of the fill composition, when expressed as free base equivalents. In any of the examples as suitable, the amount of surfactant present in the fill formulation may be from 10 to 96wt%, may be from 15 to 75wt%, and may be from 25 to 65wt% (where the amount of fill formulation includes the mass of the active ingredient, lipophilic counterion, the lipid vehicle and any other optional excipients). In any of the examples, as suitable, the amount of cosurfactant present in the fill formulation may be from 0 or 0.1 to 60wt%, may be from 5 to 50wt%, and may be from 10 to 40wt% of the fill formulation. In any of the examples as suitable, the amount of oil present in the fill formulation may be from 0 or 0.1 to 60wt%, may be from 5 to 50wt%, and may be from 10 to 45wt%.

In any of the examples, as suitable, the lipid vehicle may comprise, consist of or consist essentially of a surfactant and an optional cosurfactant, a fill formulation consisting essentially of an active ingredient (in free base equivalents) in an amount of 2 to 40wt%, a lipophilic counterion in an amount of from 2 to 40wt%, a surfactant in an amount of from 10 to 96wt%, and a cosurfactant in an amount of from 0 or 0.1 to 60wt%. In any of the examples as suitable, the fill formulation may comprise, consist of or consist essentially of an active ingredient (in free base equivalents) in an amount of 4 to 30wt%, a lipophilic counterion in an amount of from 4 to 30wt%, a surfactant in an amount of from 15 to 75wt%, and a cosurfactant in an amount of from 5 to 50wt%. In certain examples the fill formulation consists essentially of an active ingredient (in free base equivalents) in an amount of 6 to 25wt%, a lipophilic counterion in an amount of from 5 to 20wt%, a surfactant in an amount of from 25 to 65wt%, and a cosurfactant in an amount of from 10 to 40wt%. In some examples, the active ingredient is sildenafil and the lipophilic counterion is docusate.

In any of the examples the lipid vehicle may comprise, consist of or consist essentially of a surfactant, an optional cosurfactant and an optional oil, a fill formulation consisting essentially of an active ingredient (in free base equivalents) in an amount of 2 to 40wt%, a lipophilic counterion in an amount of from 2 to 40wt%, a surfactant in an amount of from 10 to 60wt%, a cosurfactant in an amount of from 0 or 0.1 to 60wt%, and an oil in an amount of from 0 or 0.1 to 60wt%.

In any of the examples the fill formulation may comprise, consist of or consist essentially of an active ingredient (in free base equivalents) in an amount of 5 to 30wt%, a lipophilic counterion in an amount of from 4 to 30wt%, a surfactant in an amount of from 15 to 50wt%, a cosurfactant in an amount of from 5 to 50wt%, and an oil in an amount of from 5 to 50wt%. In other examples the fill formulation comprises, consists essentially of or consists of an active ingredient (in free base equivalents) in an amount of 6 to 25wt%, a lipophilic counterion in an amount of from 5 to 20wt%, a surfactant in an amount of from 20 to 40wt%, a cosurfactant in an amount of from 15 to 40wt%, and an oil in an amount of from 15 to 40wt%. In other examples in an in vitro dissolution test in which the dissolution medium is 900 ml 0.01N HCl at 37°C performed using USP apparatus 11 at 100 rpm using capsule sinkers, at least 60wt% of the active ingredient is dissolved after 30 minutes. In certain examples the composition provides a Tmax of less than three hours. In certain examples the composition provides a Tmax of less than two hours. In other examples the composition provides a Tmax of less than 1 hour.

In any of the examples the composition may provide an area under the curve (AUC) that is between 75 - 125% of the area under the curve generated by a reference dosage form containing the active but free from the lipophilic counterion. In certain examples the active ingredient is sildenafil and the lipophilic counterion is docusate. In other examples the active ingredient is sildenafil and the lipophilic counterion is lauryl sulfate. In certain examples the composition comprises sildenafil docusate. In other examples the composition comprises sildenafil lauryl sulfate. In other examples the active ingredient is vardenafil and the lipophilic counterion is docusate. In other examples the composition comprises vardenafil lauryl sulfate.

Also disclosed are methods of use comprising administering to a patient in need thereof a single dosage form comprising a composition of any of the disclosed examples. In certain examples the dosage form is used to treat erectile dysfunction.

It is to be understood that both the foregoing general description and the following detailed description are exemplary and explanatory only and are not restrictive of the claimed subject matter.

### BRIEF DESCRIPTION OF THE DRAWING

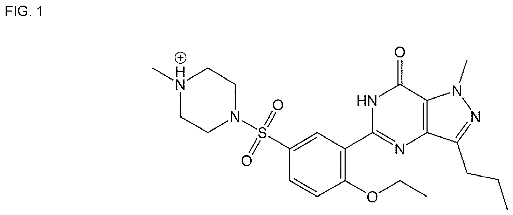

FIG. 1 is the chemical structure of sildenafil base in the ionized form.

### DETAILED DESCRIPTION

### Definitions

As used herein, reference to an element by the indefinite article "a" or "an" does not exclude the possibility that more than one of the element is present, unless the context clearly requires that there is one and only one of the elements. The indefinite article "a" or "an" thus usually means "at least one." The disclosure of numerical ranges should be understood as referring to each discrete point within the range, inclusive of endpoints, unless otherwise noted. The term "about" as used in the disclosure of numerical ranges indicates that deviation from the stated value is acceptable to the extent that the deviation is the result of measurement variability and/or yields a product of the same or similar properties. Unless otherwise indicated, all numbers expressing quantities of components, properties such as temperatures, weight percentages, and so forth, as used in the specification or claims are to be understood as being modified by the term "about." Unless otherwise indicated, implicitly or explicitly, the numerical parameters set forth are approximations that may depend on the desired properties sought, limits of detection under standard test conditions/methods, limitations of the processing method, and/or the nature of the parameter or property. When directly and explicitly distinguishing examples from discussed prior art, the examples numbers are not approximates unless the word "about" is recited.

### ORAL FORMULATIONS OF PDE5 INHIBITORS

In one aspect a composition comprises, consists essentially of or consists of a capsule and a fill formulation, wherein the fill formulation comprises a PDE5 inhibitor as the active ingredient, a lipophilic counterion to the active ingredient, the lipophilic counterion being capable of forming a lipophilic salt of the active ingredient, and a liquid, lipid vehicle in which the active ingredient is dissolved. The capsule contains the liquid fill formulation. The liquid fill formulation comprises, consists essentially of or consists of the active ingredient, lipophilic counterion, and lipid vehicle. The compositions provide high loadings of the active ingredient in the fill formulation. The compositions are capable in some examples of enabling administration of an effective amount of the active ingredient using only a single liquid filled capsule.

### CAPSULE

Capsules suitable for use in the present disclosure include any hard or soft capsule capable of containing a lipid, liquid formulation. The compositions of the present disclosure may be filled into any commercially available capsule, such as gelatin capsules, hydroxypropyl methylcellulose (HPMC) capsules, capsules made from other materials such as pullulan, starch, and pectin, and other suitable capsules as known to those skilled in the art, with the benefit of having read this disclosure. Hard capsules may be banded or sealed as is known in the art to prevent leakage.

### ACTIVE INGREDIENT

The active ingredient is a PDE5 inhibitor. In particular, the inventors have discovered that a certain subset of PDE5 inhibitors are particularly suited for examples of the disclosure. Thus, the active ingredient may be sildenafil, vardenafil, avanafil, udenafil, mirodenafil, or lodenafil. In a preferred examples, the active ingredient is sildenafil.

### LIPOPHILIC COUNTERION

The lipophilic counterion is derived from an organic acid that is capable of forming a salt with the basic active ingredient. By "capable of forming a salt" means that the basic active ingredient and the anionic lipophilic counterion, under the proper conditions as known to those skilled in the art having the benefit of this disclosure, will react to form the corresponding salt form with the active ingredient either by (i) a direct acid-base reaction between the organic acid and the basic active ingredient or (ii) a metathesis reaction (i.e., a double displacement reaction) between a salt form of the active ingredient (e.g., a hydrochloride salt) and a salt form of the lipophilic counterion (e.g., sodium salt). In some examples, at least 80 wt% of the active ingredient has reacted with the anionic lipophilic counterion to form the corresponding salt form. In other examples, at least 90 wt% of the active ingredient has reacted with the anionic lipophilic counterion to form the corresponding salt form. In yet other examples, at least 95 wt% of the active ingredient has reacted with the anionic lipophilic counterion to form the corresponding salt form. In still other examples, essentially all of the active ingredient has reacted with the anionic lipophilic counterion to form the corresponding salt form.

In certain examples the lipophilic counterion is chosen to increase the solubility of the active ingredient in the lipid vehicle. The following properties can be used to identify preferred lipophilic counterions for basic PDE5 inhibitors:
A. The counterion is an organic molecule containing carbon atoms.
B. The counterion has an acidic pKa value. The counterion preferably has at least one acidic group with a pKa value of less than 7, and more preferably at least one acidic group with a pKa value of less than 4.
C. The counterion is lipophilic. One measure of lipophilicity is the Log P or clog P of the compound. Log P refers to the log of the partition coefficient of the active ingredient in octanol-water (and clog P refers to a calculated value of Log P as is known in the art). Preferably, the Log P value is greater than 0, and more preferably is greater than 2, and may be greater than 3.
D. The counterion has a molecular weight such that the counterion:active ingredient (free base) molar mass ratio in the salt is preferably less than 2.5, and more preferably the counterion:active ingredient (free base) molar mass ratio in the salt is less than 1.5. Using the dioctylsulfosuccinate (docusate) salt of sildenafil as an example, the counterion:active ingredient molar mass ratio is 422.6 / 474.6 = 0.89.

In some examples, the lipophilic counterion comprises, consists essentially of or consists of a carboxylic acid (RC(O)O-), phosphate (ROP(O)O2-), phosphonate (RP(O)O2-), sulfonate (RS(O)2O-), sulfate (ROS(O)2O-), tetrazolyl (R-tetrazolate), bis(sulfonyl)imides (RSO2-N--SO2R) or any combination thereof where R may be any suitable group, such as an optionally substituted hydrocarbon group containing between 2 and 24 carbon atoms, and where this hydrocarbon R group is a saturated straight chained or branched hydrocarbon or a saturated cyclic hydrocarbon or an unsaturated cyclic hydrocarbon, and where the R group is unsubstituted or may be substituted by 1, 2, 3, 4, 5, or 6 or more same or different optional substituents.

In certain examples the lipophilic counterion is an alkyl sulfate, a branched alkyl sulfate, a branched alkyl sulfonate or a fatty acid.

In other examples, the lipophilic counterion is decylsulfate, lauryl sulfate, 7-ethyl-2-methyl-4-undecylsulfate, dioctylsulfosuccinate (docusate), oleate, stearate, palmitate, laurate (dodecanoate), caprate (decanoate), caprylate (octanoate) or butyl octyl sulfate.

In certain examples, the lipophilic counterion is lauryl sulfate or dioctylsulfosuccinate (docusate).

In any of the examples, the composition may comprise at least 80% of the stoichiometric amount of the anionic lipophilic counterion necessary to form a salt form with the active ingredient or other examples at least 90% of the stoichiometric amount of the anionic lipophilic counterion necessary to form a salt form of the active ingredient, or other examples at least 100% of the stoichiometric amount of the anionic lipophilic counterion necessary to form a salt form of the active ingredient.

### LIPID VEHICLE

The lipid vehicle is designed to deliver the lipophilic salt of the active in the dissolved form in the form of a liquid filled capsule. A preferred lipid vehicle is liquid at 25°C or predominantly liquid at 25°C (where the formulation is free flowing at this temperature, but where there may be some solid particles of excipient in the lipid vehicle). By predominantly liquid is meant that the amount of solids in the lipid vehicle (e.g., not including the active ingredient or lipophilic counterion) is less than 10wt% of the total weight of the lipid vehicle. The amount of solids in the lipid vehicle may be less than 5wt% of the total weight of the lipid vehicle, or may be less than 2wt% of the total weight of the lipid vehicle. In some examples, the lipid vehicle is completely liquid (e.g., no solid components) at the temperature used to fill the fill formulation into the capsule. In some examples, the lipid vehicle is liquid at 35°C, or is liquid at 40°C, or is liquid at 50°C.

While the lipophilic salt of the active may be incorporated into the lipid vehicle at temperatures above ambient conditions (e.g., 25°C), an optimal formulation is one that does not show any phase- separation or precipitation of the active (i.e., signs of physical instability) from the formulation over prolonged time-periods at 25°C, for example, a minimum of 3 months storage.

The lipid vehicle comprises a surfactant, optionally a cosurfactant; and optionally an oil. In another example, the lipid vehicle comprises a cosolvent. Other properties of the lipid vehicle include the ability to rapidly disperse in aqueous fluids (e.g., gastric fluid) on rupture and release from a capsule. It is further preferable in certain examples that the lipid vehicle maintains the active in solution, particularly in the stomach and in the small intestine, for example, by formation of an emulsion, nanoemulsion or microemulsion. It is further advantageous in certain examples that the lipid vehicle does not negatively impact the physical properties of the capsule shell, namely its capacity to rapidly dissolve and rupture in the gastro-intestinal tract or its overall physical integrity.

The lipophilic counterion and lipid vehicle are chosen so as to achieve a relatively high solubility of the active ingredient in the lipid vehicle. The active ingredient is substantially completely or is completely dissolved in the lipid vehicle, meaning that the active is homogenously dispersed in the vehicle at the molecular level (no amorphous or crystalline active particles are present). Dissolution into the lipid vehicle may be evaluated by adding the active ingredient to the lipid vehicle at the applicable wt%, followed by mixing at 30°C (or another appropriate temperature) until polarized light microscopy of removed samples confirm the absence of any undissolved active, i.e., complete incorporation of the active.

### SURFACTANT

The surfactant (or surfactant mixture) is chosen to dissolve the lipophilic salt of the active and to solubilize the lipophilic salt of the active on dispersion in aqueous fluids. Where the formulation also contains an oil, the surfactant is included to also function as an emulsifier, and to function synergistically with the oil in dissolving the lipophilic salt of the active when undiluted and in the gastro-intestinal fluids. Similarly, where the formulation also contains a cosurfactant, the surfactant and cosurfactant also work synergistically in dissolving the lipophilic salt of the active when undiluted and in the gastrointestinal fluids, and in emulsifying the oil component of the vehicle if present.

In certain examples the surfactant has the following properties: non-ionic; a hydrophilic-lipophilic balance greater than 8. The surfactant may be polyoxyethylene castor oils and/or derivatives thereof, polyoxyethylene sorbitan fatty acid esters, a mixture of (i) polyoxyethylene mono- and di-esters of C8-C22 fatty acids and (ii) glyceryl mono-, di-, and tri-esters of C8-C22 fatty acids, polyoxyethylene fatty acid esters, Vitamin E TPGS and/or derivatives thereof, polyoxyethylene-polyoxypropylene copolymers or any combination thereof.

Polyoxyethylene castor oils and derivatives may be polyoxyl 35 castor oil, polyoxyl 40 hydrogenated castor oil, or polyoxyl 60 hydrogenated castor oil, and are sold undertradenames such as as Kolliphor®, Etocas™, Croduret™.

Polyoxyethylene sorbitan fatty acid esters may be polysorbate 20, polysorbate 40, polysorbate 60, polysorbate 80, or polysorbate 85 sold, and are sold under tradenames such as Tween®, and Montanox®.

Polyoxyethylene mono- and di-esters of C8-C22 fatty acids and glycerol mono-, di-, and tri esters of C8-C22 fatty acids include caprylocaproyl macrogol-8 glycerides, oleoyl macrogol-6 glycerides, linoleoyl macrogol-6 glycerides, lauroyl macrogol-32 glycerides, stearoyl macrogol-32 glycerides and macrogol stearate, and are sold under tradenames such as Labrasol®, Labrafil®, Acconon®and Gelucire®.

Polyoxyethylene fatty acid esters include but are not limited to polyoxyl 15 hydroxystearate, polyoxyl 8 stearate, polyoxyl 40 stearate and polyoxyl 40 oleate, sold undertradenames such as Myrj™ and Kolliphor® HS-15.

Polyoxyethylene-polyoxypropylene copolymers include, but are not limited to poloxamer 124, poloxamer 188, poloxamer 407 sold undertradenames such as Pluronic® or Lutrol® or SynperonicTM.

The amount of surfactant present in the fill formulations may be from 10 to 96wt%, may be from 15 to 75wt%, and may be from 25 to 65wt% (where the amount of fill formulation includes the mass of the active ingredient, lipophilic counterion, the lipid vehicle and any other optional excipients).

### COSURFACTANT

The optional cosurfactant is chosen to work synergistically with the surfactant in dissolving the lipophilic salt of the active and to solubilize the lipophilic salt of the active on dispersion in aqueous fluids In general, the cosurfactant has the following properties: are non-ionic and a hydrophilic-lipophilic balance between 1 and 8. Exemplary cosurfactants include: propylene glycol mono- and di-esters of C8-C22 fatty acids, such as, but not limited to, propylene glycol monocaprylate, propylene glycol dicaprolate/dicaprate, propylene glycol monolaurate, sold under tradenames such as Capryol™ 90, Lauroglycol™ 90, Labrafac® PG, Capmul®; and sorbitan fatty acid esters such as, but not limited to, sorbitan monolaurate, sorbitan monopalmitate, sorbitan monooleate, sorbitan trioleate, sold under tradenames such as Span® and Montane®.

The amount of cosurfactant present in the fill formulation may be from 0 to 60wt%, or from 0.5 to 60wt%, or may be from 5 to 50wt%, or may be from 10 to 40wt% of the fill formulation.

### OIL

The optional oil is chosen to work synergistically with the surfactant and cosurfactant (if present) to dissolve the lipophilic salt of the active and to solubilize the lipophilic salt of the active on dispersion in aqueous fluids. In general, the oil has the following properties: non-ionic, largely immiscible with water, contains digestible ester functional groups.

Exemplary oils include: C8-C18 triglycerides including but not limited to almond oil, babassu oil, blackcurrant seed oil, borage oil, canola oil, castor oil, coconut oil, cod liver oil, corn oil, cottonseed oil, evening primrose oil, fish oil, grape seed oil, mustard seed oil, olive oil, palm kernel oil, palm oil, peanut oil, rapeseed oil, safflower oil, sesame oil, shark liver oil, soybean oil, sunflower oil, walnut oil, wheat germ oil, avocado oil, bran oil, hydrogenated castor oil, hydrogenated coconut oil, hydrogenated cottonseed oil, hydrogenated palm oil, hydrogenated soybean oil, partially hydrogenated soybean oil, hydrogenated vegetable oil, caprylic/capric glycerides, fractionated triglycerides, glyceryl tricaprate, glyceryl tricaproate, glyceryl tricaprylate, glyceryl tricaprylate/caprate, glyceryl tricaprylate/caprate, glyceryl tricaprylate/caprate/laurate, glyceryl tricaprylate/caprate/linoleate, glyceryl tricaprylate/caprate/stearate, glyceryl trilaurate, and C8-C18 fatty acid mono-, di and/or tri-ester mixtures of glycerol including but not limited to glyceryl linoleate, glyceryl oleate, glyceryl stearate, glyceryl caprylate, glyceryl caprate and any combinations thereof. The latter group of oils are sold under various tradenames such as Peceol™, Maisine TM, Geleol®, Capmul® and Imwitor®.

The amount of oil present in the fill formulation may be from 0 to 60wt%, may be from 0.5 to 60wt%, may be from 5 to 50wt%, or may be from 10 to 45wt%.

### COSOLVENT

In another example, the lipid vehicle comprises a co-solvent. A co-solvent is a water-soluble organic solvent. Because cosolvents are often miscible with surfactants, cosurfactants and surfactant/oil or surfactant/oil blends, they can be used to increase drug solubility in lipid-based vehicles or to facilitate the dispersion of the lipid vehicle on contact with aqueous fluids in the GI tract. A cosolvents can also be used as the lipid-vehicle itself in the absence of surfactants, cosurfactants or oils. Exemplary co- solvents include propylene carbonate, triacetin, glycerol, propylene glycol, polythethylene glycols such as PEG 400, glycofurol, ethanol, diethylene glycol monoethyl ether, oleic acid, N-methyl pyrrolidone, ethyl lactate, and triethyl citrate.
The amount of co-solvent present may vary depending on the active ingredient, the lipophilic counterion, and the other materials, if any, present in the fill formulation. In some examples, the fill formulation contains less than or equal to 10 wt % co-solvent, such as less than or equal to 7 or 5 or 2 or 1% co- solvent. In still further examples the lipid formulation or lipid vehicle contains no co-solvent. In some examples, the co-solvent is present in an amount of at least 10%, may be present in an amount of 50%, and may be present in an amount of 96%. In one example, the fill formulation consists essentially of the active ingredient, the lipophilic counterion, and the co-solvent. In some examples, the lipid vehicle contains one or more oils or lipids, without additional surfactants, co-surfactants or co-emulsifiers, or co-solvents, that is to say consists essentially of one or more oils orlipids. In some further examples the lipid vehicle contains one or more oils or lipids together with one or more surfactants, optionally together with one ormore co-solvents. In some further examples, the lipid vehicle contains one or more oils or lipids together with one or more water-soluble surfactants, optionally together with one ormore co-solvents. In some examples, the lipid vehicle contains a mixture of oil/lipid, surfactant and co-solvent. In some examples, the lipid vehicle is consists essentially of one or more surfactants/co- surfactants/co-emulsifiers, and/or solvents/co-solvents.

### OTHER OPTIONAL EXCIPIENTS

Other optional excipients added to the lipid vehicle include anti-oxidants to minimize chemical degradation of the active and/or lipid vehicle. Example antioxidants include but are not limited to vitamin E, tocopheryl polyethylene glycol succinate (TPGS), rosemary extract, ascorbic acid, ascorbyl palmitate, butylated hydroxyanisole (BHA), butylated hydroxytoluene (BHT), or any combination thereof.

The amount of antioxidant in the lipid vehicle may be from 0 to 5wt% or from 0.1 to 5wt%.

Other excipients such as thickeners or dyes may be present in relatively minor amounts so long as such excipients do not adversely affect the solubility of the active ingredient in the lipid vehicle. In some examples, such excipients make up less than 5wt% of the lipid vehicle, and may be present in an amount of less than 2wt% of the lipid vehicle.

### EXEMPLARY LIPID VEHICLES

In some examples in which the lipid vehicle comprises, consists of or consists essentially of a surfactant and an optional cosurfactant, a fill formulation includes or consists essentially of an active ingredient (in free base equivalents) in an amount of 2 to 40wt%, a lipophilic counterion in an amount of from 2 to 40wt%, a surfactant in an amount of from 10 to 96wt%, and a cosurfactant in an amount of from 0 to 60wt% or 0.1 to 60wt%. In other examples, a fill formulation comprises, consists of or consists essentially of an active ingredient (in free base equivalents) in an amount of 4 to 30wt%, a lipophilic counterion in an amount of from 4 to 30wt%, a surfactant in an amount of from 15 to 75wt%, and a cosurfactant in an amount of from 5 to 50wt%. In other examples, a fill formulation comprises, consists of or consists essentially of an active ingredient (in free base equivalents) in an amount of 6 to 25wt%, a lipophilic counterion in an amount of from 5 to 20wt%, a surfactant in an amount of from 25 to 65wt%, and a cosurfactant in an amount of from 10 to 40wt%. In certain examples, the active ingredient is sildenafil and the lipophilic counterion is docusate.

In some examples in which the lipid vehicle includes a surfactant an optional cosurfactant and an optional oil, a fill formulation comprises, consists of or consists essentially of an active ingredient (in free base equivalents) in an amount of 2 to 40wt%, a lipophilic counterion in an amount of from 2 to 40wt%, a surfactant in an amount of from 10 to 60wt%, a cosurfactant in an amount of from 0 to 60wt% or 0.1 to 60wt%, and an oil in an amount of from 0 to 60wt% or 0.1 to 60wt%. In other examples, a fill formulation comprises, consists of orconsists essentially of an active ingredient (in free base equivalents) in an amount of 5 to 30wt%, a lipophilic counterion in an amount of from 4 to 30wt%, a surfactant in an amount of from 15 to 50wt%, a cosurfactant in an amount of from 5 to 50wt%, and an oil in an amount of from 5 to 50wt%. In other examples, a fill formulation consists essentially of an active ingredient (in free base equivalents) in an amount of 6 to 25wt%, a lipophilic counterion in an amount of from 5 to 20wt%, a surfactant in an amount of from 20 to 40wt%, a cosurfactant in an amount of from 15 to 40wt%, and an oil in an amount of from 15 to 40wt%. In any of the disclosed examples, the active ingredient may be sildenafil and the lipophilic counterion is docusate.

### ACTIVE LOADING IN FILL FORMULATION

Examples of the compositions are capable of achieving high loadings of active ingredient in the fill formulations. The lipophilic counterion and lipid vehicle are chosen so that the lipid vehicle is capable of dissolving a substantial amount of active ingredient. The suitability of the various lipid vehicle components may be determined by evaluating the solubility of the lipophilic salt of the active in surfactants, cosurfactants and oils. The lipid vehicle may then be chosen to provide sufficient solubility of the active in the lipid vehicle. For example, solubilities of various lipophilic salts of sildenafil in various surfactants, cosurfactants, oils and cosolvents are given below:

| **Excipient** | **Solubility in mg/g as sildenafil free base equivalents, at 30°C** | | |
|---|---|---|---|
| | **Sildenafil free base** | **Sildenafil lauryl sulfate** | **Sildenafil docusate** |
| Soybean oil | <10 | | |
| Miqlvol® 812 | <10 | 25-50 | 50-100 |
| Maisine™ 35-1 | <10 | | |
| Imwitor® 308 | 10-20 | 100-200 | >200 |
| Lauroqlycol™ 90 | <10 | | |
| Capryol TM 90 | 10-20 | 100-200 | >200 |
| Labrasol® | 10-20 | 100-200 | >200 |
| Kolliphor® EL | <10 | | |
| PEG 400 | 10-20 | 100-200 | >200 |

From the above solubility results, a lipid vehicle may be designed initially based on components that have a capacity to dissolve the target dose of the active in no more than 1 gram, for example, a target solubility of 100 mg/g. To achieve the delivery of the target dose with a smaller amount of lipid vehicle, it is advantageous to select components that dissolve the target dose at higher concentrations, for example at 200 mg/g. Such concentrations are possible using lipophilic salt forms of sildenafil, but not when using the free base form.

The resulting compositions achieve relatively high loadings of the active ingredient in the fill formulation when using lipophilic salt forms of the active. In some examples, the amount of active ingredient (free base equivalent) present in the composition is at least 2.5wt% of the fill formulation. In other examples, the amount of active ingredient (free base) present in the composition is at least 5wt% of the fill formulation, may be at least 7.5wt% of the fill formulation, and may be at least 10wt% of the fill formulation, and may be at least 15wt% of the formulation, and may be at least 20wt% of the formulation.

In other examples, the mass of the fill formulation is less than 800 mg and the amount of active ingredient (free base equivalent) in the composition is at least 25mg, may be at least 50mg, may be at least 75mg, and may be at least 100mg when using the lipophilic salt form of the active.

In other examples, the mass of the fill formulation is less than 600mg and the amount of active ingredient (free base equivalent) in the composition is at least 25mg, may be at least 50mg, may be at least 75mg, and may be at least 100mg when using the lipophilic salt form of the active.

In other examples, the mass of the fill formulation is less than 450mg and the amount of active ingredient (free base equivalent) in the composition is at least 25mg, may be at least 50mg, may be at least 75mg, and may be at least 100mg when using the lipophilic salt form of the active.

Key test outcomes include (i) miscibility of the different components to form a single phase vehicle (ii) a capacity to fully dissolve the target dose of the active and/or (iii) performance indicators such as immediate release from capsules and ongoing solubilization of the active.

### PERFORMANCE

In some examples, the compositions are capable of providing immediate release of the active ingredient from the capsule and ongoing good solubilization. These properties may be evaluated in an in vitro dissolution test in which the dissolution medium is, for example, 900 ml 0.01 N HCl at 37°C performed using USP apparatus II at 100 rpm using capsule sinkers. In testing lipid formulations, it is optional to also include a small amount (<5%) of suitable surfactant in the dissolution medium, such as sodium lauryl sulfate or polysorbate 80. In some examples, when the composition is evaluated in such a dissolution test, at least 60wt% of the active ingredient is dissolved after 30 minutes. In other examples, when the composition is evaluated in such a dissolution test, at least 80wt% of the active ingredient is dissolved after 30 minutes.

In some examples, the compositions provide relatively fast onset in vivo. Time of onset may be evaluated in animal or human studies and measuring the time to maximum concentration of active ingredient in the blood (e.g., Tmax). In some examples, the composition provides a Tmax of less than three hours. In some examples, the composition provides a Tmax of less than two hours. In other examples, the composition provides a Tmax of less than 1 hour.

Other in vivo markers of performance in humans or in animals include the overall extent of drug absorption, which is often measured by the blood (serum or plasma) versus time area under the curve (AUC) value. In some examples, the compositions provide AUC values that are within 50 - 150% of the AUC value generated by the reference dosage form (which may be a commercial tablet dosage form, or another dosage form containing the free or commercial salt form). In some examples, the compositions provide AUC values that are within 75 - 125% of the AUC value generated by the reference dosage form.

### METHOD OF USE

Examples of the compositions disclosed herein may be used to treat any indication for which administration of a PDE5 inhibitor is indicated. Examples of a method are provided for treating erectile dysfunction by administering a composition as disclosed herein. In other examples, a method is provided for treating erectile dysfunction by administering a single liquid filled capsule containing a composition as disclosed herein. In other examples a method is provided for treating pulmonary hypertension by administering a composition as disclosed herein. In other examples, a method is provided for treating pulmonary hypertension by administering a single liquid filled capsule containing a composition as disclosed herein.

It should be understood that the examples described herein are not limited thereto. Other examples of the present disclosure will be apparent to those skilled in the art from consideration of the specification and practice of the disclosed examples. The following examples should be considered as exemplary only, with a true scope of the present disclosure being defined by the claims appended hereto.

### Example 1 - Salt Preparation

Several salts of sildenafil were prepared as follows.

Sildenafil HCl preparation: Sildenafil free base (5.0g, 10.5 mmol) in 250 ml anhydrous diethylether was added 5.3 ml HCl in diethylether (2.0M solution), resulting solution stirred for 4 hours, filtered and washed with cold portions of diethylether to give the desired product as a white solid (5.33g, 99%).

Sildenafil docusate: Sildenafil HCl (1.53g, 3.0 mmol) and sodium docusate (1.33g, 3.0 mmol) were weighed into a round bottom flask and suspended in ethyl acetate (100ml) / water (50ml) and stirred for 3 hours. The resulting mixture was transferred to a separating funnel and the organic layer was separated, the aqueous layer washed with a further 2 x 100ml ethyl acetate, the combined organics were backwashed with distilled water (50ml portions) until negative to a silver nitrate precipitate test. The solution was dried (sodium sulfate= Na2SO4), filtered and concentrated in vacuo to give the desired product as a white solid (2.61g, yield= 97%), this product could be crystallized by dissolution in diethylether followed by precipitation with petroleum spirits. The resulting crystalline powder of sildenafil docusate had melting point of 70.9°C (in contrast, the melting temperature of the sildenafil free base is about 195°C). Counterion cLog P = 5.96.

Sildenafil lauryl sulfate: Sildenafil HCI (1.54g, 3.0 mmol) and sodium lauryl sulfate (869mg, 3.0 mmol) were weighed into a round bottom flask and suspended in ethyl acetate (100ml) / water (50ml) and stirred for 4 hours. The resulting mixture was transferred to a separating funnel and the organic layer was separated, the aqueous layer washed with a further 2 x 100ml ethyl acetate, the combined organics were backwashed with distilled water (50ml portions) until negative to a silver nitrate precipitate test. The solution was dried (Na2SO4), filtered and concentrated in vacuo to give the desired product as a white solid (2.19g, 98%), this product could be crystallized by dissolution in ethyl acetate followed by precipitation with petroleum spirits. The resulting crystalline powder of sildenafil lauryl sulfate had melting point of between 103-118°C (in contrast, the melting temperature of the sildenafil free base is about 195°C). Counterion cLog P = 5.39.

Sildenafil 7-ethyl-2-methylundecan-4-yl sulfate: Sildenafil HCl (345mg, 0.7 mmol) was weighed into a round bottom flask and suspended in ethyl acetate (30ml) / water (10ml), 0.74ml Niaproof-4® soln (∼27% in water) stirred for 3 hours. The resulting mixture was transferred to a separating funnel and the organic layer was separated, the aqueous layer washed with a further 2 x 30ml ethyl acetate, the combined organics were backwashed with distilled water (10ml portions) until negative to a silver nitrate precipitate test. The solution was dried (Na2SO4), filtered and concentrated in vacuo to give the desired product as a white solid (493mg, 95%), which was further purified by diethyl ether trituration. Counterion cLog P = 5.9.

### Example 2

Fill compositions were prepared by first preparing a lipid vehicle in a glass vial having the following components, and then adding the lipophilic salt of the active ingredient to the lipid vehicle. The resulting fill formulation was then mixed at 30 to 40°C until the active ingredient completely dissolved.
Complete dissolution of the active ingredient was confirmed using a polarized light microscope to confirm the absence of drug crystals.

| **%w/w** | **Component** | **Mass (mg) for 50 mg Sildenafil Capsule** | **Mass (mg) for 100 mg Sildenafil Capsule** |
|---|---|---|---|
| 24.0 | Sildenafil docusate | 94.5 | 188.9 |
| 15.2 | Propylene glycol monocaprylate (Capryol™ 90) | 59.5 | 119.6 |
| 30.4 | PEG-8 caprylic/capric glycerides (Labrasol®) | 119.7 | 239.3 |
| 30.4 | Polyoxyl 35 castor oil (Kolliphor® EL) | 119.7 | 239.3 |
| 100.0 | | 393.8 | 787.1 |

Inthis example, propylene glycol monocaprylate is the cosurfactant, PEG-8 caprylic/capric glycerides and polyoxyl 35 castor oil are both surfactants.

The fill formulation was evaluated for physically stability (in closed vials) at 25°C/65% RH for 3 months and found to be physically stable.

### Example 3

A fill formulation was prepared as in Example 2. Complete dissolution of the active ingredient was confirmed using a polarized light microscope to confirm the absence of drug crystals.

| **%w/w** | **Component** | **Mass (mg) for 50 mg Sildenafil Capsule** | **Mass (mg) for 100 mg Sildenafil Capsule** |
|---|---|---|---|
| 24.0 | Sildenafil docusate | 94.5 | 188.9 |
| 15.2 | Medium-chain triglycerides (Miglyol® 812) | 59.5 | 119.6 |
| 30.4 | Propylene glycol monocaprylate (Capryol™ 90) | 119.7 | 239.3 |
| 30.4 | Polyoxyl 35 castor oil (Kolliphor® EL) | 119.7 | 239.3 |
| 100.0 | | 393.8 | 787.1 |

Inthis example, medium-chain triglycerides is the oil, propylene glycol monocaprylate is the cosurfactant and polyoxyl 35 castor oil is the surfactant.

The fill formulation was evaluated for physically stability (in closed vials) at 25°C/65% RH for 3 months and found to be physically stable.

### Example 4

| **%w/w** | **Component** | **Mass (mg) for 50 mg Sildenafil Capsule** | **Mass (mg) for 100 mg Sildenafil Capsule** |
|---|---|---|---|
| 34.0 | Sildenafil docusate | 94.5 | 188.9 |
| 13.2 | Propylene glycol monocaprylate (Capryol™ 90) | 36.7 | 73.3 |
| 26.4 | PEG-8 caprylic/capric glycerides (Labrasol®) | 73.4 | 146.7 |
| 26.4 | Polyoxyl 35 castor oil (Kolliphor® EL) | 73.4 | 146.7 |
| 100.0 | | 277.9 | 555.6 |

The formulation was evaluated for physically stability (in closed vials) at 25°C/65% RH for 3 months and found to be physically stable.

### Example 5

Experiments were conducted in fasted male Sprague-Dawley rats (250-300 g) to evaluate pharmacokinetics in vivo of the compositions. A day prior to the study, rats were anesthetized with isoflurane, and the right carotid artery was surgically cannulated with polyethylene tubing to facilitate blood collection. Animals were allowed to recover overnight and were fasted up to 12 hours priorto and **4** hours after dose administration with water provided ad libitum. Sildenafil lipophilic salts were administered to rats as lipid formulations (∼280 mg of Example 2 or Example 3) uniformly dispersed in 1 ml Milli-Q water. Sildenafil citrate (control) was administered as an aqueous solution/suspension comprising 0.5% (w/v) sodium carboxymethylcellulose, 0.4% Tween® 80, and 0.9% w/v sodium chloride in water. All treatments were administered by oral gavage and the target dose was 25 mg/kg of sildenafil free base equivalent (∼6-7 mg per rat).

Results are presented in the table below:

| **N=4 ± SD** | **Cₘₐₓ (ng/ml)** | **Tₘₐₓ (h)** | **AUC (ng·h/ml)** | **Half-life (h)** |
|---|---|---|---|---|
| Sildenafil citrate aq. suspension | 515.5 ± 254.3 | 0.31 ± 0.14 | 419.4±183.6 | 0.48 ± 0.05 |
| Sildenafil docusate EXAMPLE 2 | 251.9 ± 121.6 | 0.50 ± 0.36 | 406.8 ± 168.1 | 0.65 ± 0.02 |
| Sildenafil docusate EXAMPLE 3 | 364.7 ± 168.8 | 0.46 ± 0.22 | 493.8 ± 68.6 | 0.84 ± 0.29 |

No statistical (non-paired ANOVA statistical test) differences in AUC values across treatments, highlighting good absorbability of sildenafil lipophilic salts compared with the suspension of the sildenafil citrate aqueous suspension.

### Example 6

Sildenafil dodecanoate (laurate) was prepared as follows. Sildenafil HCl (250 mg, 0.49 mmol) and sodium dodecanoate (109 mg, 0.49 mmol) were suspended in 10 ml methanol and stirred overnight at ambient temperature. The resulting suspension was concentrated in vacuo to give a residue which was suspended in 10 ml chloroform, the cloudy solution was filtered and the filtrate concentrated in vacuo to give the desired product (313 mg, yield= 95%). The resulting crystalline powder of sildenafil dodecanoate had a melting onset at about 90°C, and had completely melted by 180°C (in contrast, the melt temperature of sildenafil free base is about 195°C). Counterion cLog P = 4.48.

### Example 7

A fill composition for a capsule containing sildenafil docusate and a co-solvent was prepared as follows. PEG 400, a cosolvent, was added to a glass vial, followed by addition of sildenafil docusate in an amount such that the resulting fill composition was 24wt% sildenafil docusate and 76wt% PEG 400. The resulting fill formulation was then mixed at 30 to 40°C until the sildenafil docusate completely dissolved.

### Example 8

A fill composition for a capsule containing sildenafil docusate, an oil and a surfactant was prepared as follows. Glyceryl monocaprylate (sold under the trade name Imwitor® 308) (a lipid/oil) and Polysorbate 80 (sold under the trade name Tween 80) (a surfactant) were added to a glass vial, followed by addition of sildenafil docusate in an amount such that the resulting fill composition was 24wt% sildenafil docusate, 30.4wt% glyceryl monocaprylate, and 45.6wt% polysorbate 80. The resulting fill formulation was then mixed at 30 to 40°C until the sildenafil docusate completely dissolved.

### Example 9

Vardenafil docusate was prepared as follows. Vardenafil HCl (525 mg, 1.0 mmol) and sodium docusate (444 mg, 1.0 mmol) were weighed into a round bottom flask and dissolved in ethyl acetate (10ml) / water (10ml) and stirred for 4 hours. The resulting mixture was transferred to a separating funnel and the organic layer was separated, the aqueous layer washed with a further 2 x 10ml ethyl acetate, the combined organics were backwashed with distilled water (10 ml portions) until negative to a silver nitrate precipitate test. The solution was dried (sodium sulfate= Na2SO4), filtered and concentrated in vacuo and dried under high vacuum to give the desired product as an amorphous solid (893 mg, yield= 98%). The resulting amorphous powder of vardenafil docusate had a solid-to-liquid transition between 45-65°C and a measured glass transition temperature of -20°C (in contrast, the melting temperature of the vardenafil base is about 192°C).

### Example 10

Vardenafil lauryl sulfate was prepared as follows. Vardenafil HCl (539 mg, 1.03 mmol) and sodium lauryl sulfate (296 mg, 1.03 mmol) were weighed into a round bottom flask and dissolved in ethyl acetate (10ml) / water (10ml) and stirred for 3 hours. The resulting mixture was transferred to a separating funnel and the organic layer was separated, the aqueous layer washed with a further 2 x 10ml ethyl acetate, the combined organics were backwashed with distilled water (10ml portions) until negative to a silver nitrate precipitate test. The solution was dried (sodium sulfate= Na₂SO₄), filtered and concentrated in vacuo to give the desired product as a white solid (731 mg, yield= 98%). The resulting amorphous powder of vardenafil lauryl sulfate had a solid-to-liquid transition between 70-85°C (in contrast, the melting temperature of the vardenafil base is about 192°C). This product could be crystallised from ethyl acetate.

### Example 11

Vardenafil dodecanoate (laurate) was prepared as follows. Vardenafil HCl (250 mg, 0.48 mmol) and sodium dodecanoate (106 mg, 0.48 mmol) were dissolved in 10 ml methanol and stirred overnight at ambient temperature. The resulting solution was concentrated in vacuo to give a residue which was suspended in 10ml chloroform, the cloudy solution was filtered and the filtrate concentrated in vacuo to give the desired product (315mg, yield = 96%). The resulting crystalline powder of vardenafil dodecanoate had a melting onset of -95°C and complete melting by 175°C (in contrast, the melting temperature of the vardenafil base is about 192°C). Counterion cLog P = 4.48.

### Example 12

Vardenafil dodecyl sulfonate: Vardenafil HCl (400 mg, 0.76 mmol) and sodium dodecyl sulfonate (207 mg, 0.76 mmol) were dissolved in 10ml methanol and stirred overnight at ambient temperature. The resulting solution was concentrated in vacuo to give a residue which was suspended in 1:1 chloroform: methanol (10 ml), the cloudy solution was filtered and the filtrate concentrated in vacuo to give the desired product (507mg, yield= 90%). The resulting crystalline powder of vardenafil dodecyl sulfonate had a melting onset of -130°C and complete melting by 165°C (in contrast, the melting temperature of the vardenafil base is about 192°C). Counterion cLog P = 4.07.

### Example 13

A fill composition was prepared by first preparing a lipid vehicle in a glass vial having the following components: Propylene glycol monocaprylate (sold under the trade name Capryol™ 90), PEG-8 caprylic/capric glycerides (sold under the trade name Labrasol®), and polyoxyl 35 castor oil (sold under the trade name Kolliphor® EL). Vardenafil docusate was then added to the lipid vehicle, in an amount such that the fill composition was 24.0wt% vardenafil docusate, 15.2 wt% propylene glycol monocaprylate, 30.4 wt% PEG-8 caprylic/capric glycerides, and 30.4 wt% polyoxyl 35 castor oil. The resulting fill formulation was then mixed at 30 to 40°C until the vardenafil docusate was completely dissolved.

### Example 14

A fill composition was prepared by first preparing a lipid vehicle in a glass vial having the following components: Propylene glycol monocaprylate (sold under the trade name Capryol™ 90), PEG-8 caprylic/capric glycerides (sold under the trade name Labrasol®), and polyoxyl 35 castor oil (sold under the trade name Kolliphor® EL). Vardenafil dodecanoate was then added to the lipid vehicle, in an amount such that the fill composition was 3.5wt% vardenafil dodecanoate, 19.3 wt% propylene glycol monocaprylate, 38.6 wt% PEG-8 caprylic/capric glycerides, and 38.6 wt% polyoxyl 35 castor oil. The resulting fill formulation was then mixed at 30 to 40°C until the vardenafil dodecanoate was completely dissolved.

In view of the many possible examples to which the principles of the disclosure may be applied, it should be recognized that the illustrated examples are only preferred examples and should not be taken as limiting examples.

## Claims

1. A composition comprising a capsule and a fill formulation, wherein the fill formulation comprises:
a. an active ingredient selected from sildenafil, vardenafil, avanafil, udenafil, mirodenafil and lodenafil;
b. a lipophilic counterion to the active ingredient, the lipophilic counterion being present in an amount of at least 90 mol% of the active ingredient so as to be capable of forming a lipophilic salt of the active ingredient; and
c. a lipid vehicle that is liquid or predominantly liquid at25°C;
wherein the active ingredient is completely dissolved in the lipid vehicle in an amount of at least 1.0wt% at 25°C (expressed as free base equivalents) of the fill formulation.

2. The composition of any of the preceding claims wherein the active ingredient is present in the composition in an amount of at least 2.5wt% (expressed as free base equivalents).

3. The composition of any of the preceding claims wherein the lipid vehicle comprises:
a) a surfactant or a mixture of surfactants;
b) optionally a cosurfactant; and
c) optionally an oil.

4. The composition of any of the preceding claims wherein said lipid vehicle comprises a co-solvent.

5. The composition of any of the preceding claims wherein the surfactant or surfactants are:
a) polyoxyethylene sorbitan fatty acid esters;
b) a mixture of (i) polyoxyethylene mono- and di-esters of C₈-C₂₂ fatty acids and (ii) glyceryl mono-, di-, and tri-esters of C8-C22 fatty acids;
c) polyoxyethylene castor oils and derivatives;
d) polyoxyethylene fatty acid esters
e) Vitamin E TPGS and derivatives thereof;
f) polyoxyethylene-polyoxypropylene copolymers, or
g) any combination thereof.

6. The composition of any of the preceding claims wherein the cosurfactant is selected from the group consisting of: propylene glycol mono- and di-esters of C₈-C₂₂ fatty acids; and sorbitan fatty acid esters.

7. The composition of any of the preceding claims wherein the oil comprises a C₈-C₁₈ fatty acid ester of glycerol.

8. The composition of any of the preceding claims wherein when the active ingredient is sildenafil the lipophilic salt of the active ingredient has a solubility in the lipid vehicle that is at least 5-fold greater than the solubility of the citrate salt form of the active ingredient in the lipid vehicle, and when the active ingredient is vardenafil the lipophilic salt of the active ingredient has a solubility in the lipid vehicle that is at least 5-fold greater than the solubility of the hydrochloride salt form of the active ingredient in the lipid vehicle.

9. The composition of any of the preceding claims wherein the counterion has at least one acidic group with a pKa value of less than 7, and more preferably at least one acidic group with a pKa value of less than 4.

10. The composition of any of the preceding claims wherein the counterion has a molecular weight such that the counterion:active ingredient molar mass ratio in the salt is preferably less than 2.5, and more preferably the counterion:active ingredient molar mass ratio in the salt is less than 1.5.

11. The composition of any of the preceding claims wherein the lipophilic counterion is decylsulfate, lauryl sulfate, 7-ethyl-2-methyl-4-undecylsulfate, dioctylsulfosuccinate (docusate), oleate, stearate, palmitate, laurate (dodecanoate), caprate (decanoate), caprylate (octanoate), or butyl octyl sulfate.

12. The composition of any of the preceding claims wherein the amount of surfactant present in the fill formulation is from 10 to 96wt%, or is from 15 to 75wt%, or is from 25 to 65wt%, the amount of cosurfactant present in the fill formulation is from 0 to 60wt%, is from 5 to 50wt%, or is from 10 to 40wt%, and the amount of oil present in the fill formulation is from 0 to 60wt%, or is from 5 to 50wt%, or is from 10 to 45wt% (where the amount of fill formulation includes the mass of the active ingredient, lipophilic counterion, the lipid vehicle and any other optional excipients).

13. The composition of any of the preceding claims wherein the lipid vehicle consists essentially of a surfactant and an optional cosurfactant, a fill formulation consists essentially of an active ingredient (in free base equivalents) in an amount of 2 to 40wt%, a lipophilic counterion in an amount of from 2 to 40wt%, a surfactant in an amount of from 10 to 96wt%, and a cosurfactant in an amount of from 0 or 0.1 to 60wt%.

14. The composition of any of the preceding claims comprising a co-solvent.

## Patentansprüche

1. Zusammensetzung, umfassend eine Kapsel und eine Füllformulierung, wobei die Füllformulierung Folgendes umfasst:
a. einen Wirkstoff, der aus Sildenafil, Vardenafil, Avanafil, Udenafil, Mirodenafil und Lodenafil ausgewählt ist;
b. ein lipophiles Gegenion zu dem Wirkstoff, wobei das lipophile Gegenion in einer Menge von mindestens 90 Mol-% des Wirkstoffs vorliegt, so dass es dazu fähig ist, ein lipophiles Salz des Wirkstoffs zu bilden; und
c. ein Lipidvehikel, das bei 25 °C flüssig oder überwiegend flüssig ist;
wobei der Wirkstoff in dem Lipidvehikel in einer Menge von mindestens 1,0 Gew.-% bei 25 °C (ausgedrückt als Äquivalente der freien Base) der Füllformulierung vollständig gelöst ist.

2. Zusammensetzung nach einem der vorhergehenden Ansprüche, wobei der Wirkstoff in der Zusammensetzung in einer Menge von mindestens 2,5 Gew.-% (ausgedrückt als Äquivalente der freien Base) vorliegt.

3. Zusammensetzung nach einem der vorhergehenden Ansprüche, wobei das Lipidvehikel Folgendes umfasst:
a) ein Tensid oder eine Mischung von Tensiden;
b) gegebenenfalls ein Cotensid und
c) gegebenenfalls ein Öl.

4. Zusammensetzung nach einem der vorhergehenden Ansprüche, wobei das Lipidvehikel ein Cosolvens umfasst.

5. Zusammensetzung nach einem der vorhergehenden Ansprüche, wobei es sich bei dem Tensid bzw. den Tensiden um
a) Polyoxyethylensorbitanfettsäureester;
b) eine Mischung von (i) Polyoxyethylenmono- und -diestern von C₈-C₂₂-Fettsäuren und (ii) Glycerylmono-, -di- und -triestern von C₈-C₂₂-Fettsäuren;
c) Polyoxyethylenricinusöle (Polyoxyethylene castor oils) und Derivate;
d) Polyoxyethylenfettsäureester;
e) Vitamin-E-TPGS und Derivate davon;
f) Polyoxyethylen-Polyoxypropylen-Copolymere oder
g) eine beliebige Kombination davon
handelt.

6. Zusammensetzung nach einem der vorhergehenden Ansprüche, wobei das Cotensid aus der Gruppe bestehend aus Propylenglykolmono- und -diestern von C₈-C₂₂-Fettsäuren und Sorbitanfettsäureestern ausgewählt ist.

7. Zusammensetzung nach einem der vorhergehenden Ansprüche, wobei das Öl einen C₈-C₁₈-Fettsäureester von Glycerin umfasst.

8. Zusammensetzung nach einem der vorhergehenden Ansprüche, wobei dann, wenn es sich bei dem Wirkstoff um Sildenafil handelt, das lipophile Salz des Wirkstoffs eine Löslichkeit in dem Lipidvehikel aufweist, die mindestens um den Faktor 5 größer ist als die Löslichkeit der Citratsalzform des Wirkstoffs in dem Lipidvehikel, und dann, wenn es sich bei dem Wirkstoff um Vardenafil handelt, das lipophile Salz des Wirkstoffs eine Löslichkeit in dem Lipidvehikel aufweist, die mindestens um den Faktor 5 größer ist als die Löslichkeit der Hydrochloridsalzform des Wirkstoffs in dem Lipidvehikel.

9. Zusammensetzung nach einem der vorhergehenden Ansprüche, wobei das Gegenion mindestens eine saure Gruppe mit einem pKa-Wert von weniger als 7 und weiter bevorzugt mindestens eine saure Gruppe mit einem pKa-Wert von weniger als 4 aufweist.

10. Zusammensetzung nach einem der vorhergehenden Ansprüche, wobei das Gegenion ein solches Molekulargewicht aufweist, dass das Gegenion:Wirkstoff-Molmassenverhältnis in dem Salz vorzugsweise weniger als 2,5 beträgt und weiter bevorzugt das Gegenion:Wirkstoff-Molmassenverhältnis in dem Salz weniger als 1,5 beträgt.

11. Zusammensetzung nach einem der vorhergehenden Ansprüche, wobei es sich bei dem lipophilen Gegenion um Decylsulfat, Laurylsulfat, 7-Ethyl-2-methyl-4-undecylsulfat, Dioctylsulfosuccinat (Docusat), Oleat, Stearat, Palmitat, Laurat (Dodecanat), Caprat (Decanoat), Caprylat (Octanoat) oder Butyloctylsulfat handelt.

12. Zusammensetzung nach einem der vorhergehenden Ansprüche, wobei die in der Füllformulierung vorliegende Tensidmenge 10 bis 96 Gew.-% oder 15 bis 75 Gew.-% oder 25 bis 65 Gew.-% beträgt, die in der Füllformulierung vorliegende Cotensidmenge 0 bis 60 Gew.-%, 5 bis 50 Gew.-% oder 10 bis 40 Gew.-% beträgt und die in der Füllformulierung vorliegende Ölmenge 0 bis 60 Gew.-% oder 5 bis 50 Gew.-% oder 10 bis 45 Gew.-% beträgt (wobei die Menge der Füllformulierung die Masse des Wirkstoffs, des lipophilen Gegenions, des Lipidvehikels und jeglicher anderer fakultativer Hilfsstoffe einschließt).

13. Zusammensetzung nach einem der vorhergehenden Ansprüche, wobei das Lipidvehikel im Wesentlichen aus einem Tensid und einem fakultativen Cotensid besteht, eine Füllformulierung im Wesentlichen aus einem Wirkstoff (in Äquivalenten der freien Base) in einer Menge von 2 bis 40 Gew.-%, einem lipophilen Gegenion in einer Menge von 2 bis 40 Gew.-%, einem Tensid in einer Menge von 10 bis 96 Gew.-% und einem Cotensid in einer Menge von 0 oder 0,1 bis 60 Gew.-% besteht.

14. Zusammensetzung nach einem der vorhergehenden Ansprüche, die ein Cosolvens umfasst.

## Revendications

1. Composition comprenant une capsule et une formulation de remplissage, la formulation de remplissage comprenant :
a. un ingrédient actif choisi parmi le sildénafil, le vardénafil, l'avanafil, l'udénafil, le mirodénafil et le lodénafil ;
b. un contre-ion lipophile à l'ingrédient actif, le contre-ion lipophile étant présent en une quantité d'au moins 90 % en moles de l'ingrédient actif de sorte à être capable de former un sel lipophile de l'ingrédient actif ; et
c. un véhicule lipidique qui est liquide ou principalement liquide à 25 °C ;
l'ingrédient actif étant complètement dissous dans le véhicule lipidique en une quantité d'au moins 1,0 % en poids à 25 °C (exprimée en tant qu'équivalents de base libre) de la formulation de remplissage.

2. Composition selon l'une quelconque des revendications précédentes, l'ingrédient actif étant présent dans la composition en une quantité d'au moins 2,5 % en poids (exprimée en tant qu'équivalents de base libre).

3. Composition selon l'une quelconque des revendications précédentes, le véhicule lipidique comprenant :
a) un tensioactif ou un mélange de tensioactifs ;
b) éventuellement un co-tensioactif ; et
c) éventuellement une huile.

4. Composition selon l'une quelconque des revendications précédentes, le véhicule lipidique comprenant un co-solvant.

5. Composition selon l'une quelconque des revendications précédentes, le ou les tensioactifs étant :
a) des esters d'acides gras de polyoxyéthylène sorbitane ;
b) un mélange de (i) monoesters et de diesters de polyoxyéthylène d'acides gras en C₈ à C₂₂ et (ii) de monoesters, de diesters, et de triesters de glycéryle d'acides gras en C₈ à C₂₂ ;
c) des huiles de ricin de polyoxyéthylène (polyoxyethylene castor oils) et des dérivés ;
d) des esters d'acides gras de polyoxyéthylène
e) de la vitamine E TPGS et des dérivés correspondants ;
f) des copolymères de polyoxyéthylène-polyoxypropylène, ou
g) une quelconque combinaison correspondante.

6. Composition selon l'une quelconque des revendications précédentes, le co-tensioactif étant choisi dans le groupe constitué par : des monoesters et des diesters de propylèneglycol d'acides gras en C₈ à C₂₂ ; et des esters d'acides gras de sorbitane.

7. Composition selon l'une quelconque des revendications précédentes, l'huile comprenant un ester d'acide gras en C₈ à C₁₈ de glycérol.

8. Composition selon l'une quelconque des revendications précédentes, lorsque l'ingrédient actif est le sildénafil, le sel lipophile de l'ingrédient actif possédant une solubilité dans le véhicule lipidique qui est au moins 5 fois supérieure à la solubilité de la forme de sel de citrate de l'ingrédient actif dans le véhicule lipidique, et lorsque l'ingrédient actif est le vardénafil, le sel lipophile de l'ingrédient actif possédant une solubilité dans le véhicule lipidique qui est au moins 5 fois supérieure à la solubilité de la forme de sel de chlorhydrate de l'ingrédient actif dans le véhicule lipidique.

9. Composition selon l'une quelconque des revendications précédentes, le contre-ion possédant au moins un groupe acide doté d'une valeur de pKa inférieure à 7, et plus préférablement au moins un groupe acide doté d'une valeur de pKa inférieure à 4.

10. Composition selon l'une quelconque des revendications précédentes, le contre-ion possédant un poids moléculaire tel que le rapport de masses molaires de contre-ion:ingrédient actif dans le sel est préférablement inférieur à 2,5, et plus préférablement le rapport de masses molaires de contre-ion:ingrédient actif dans le sel est inférieur à 1,5.

11. Composition selon l'une quelconque des revendications précédentes, le contre-ion lipophile étant le décylsulfate, le laurylsulfate, le 7-éthyl-2-méthyl-4-undécylsulfate, le dioctylsulfosuccinate (docusate), l'oléate, le stéarate, le palmitate, le laurate (dodécanoate), le caprate (décanoate), le caprylate (octanoate), ou le butyloctylsulfate.

12. Composition selon l'une quelconque des revendications précédentes, la quantité de tensioactif présent dans la formulation de remplissage étant de 10 à 96 % en poids, ou étant de 15 à 75 % en poids, ou étant de 25 à 65 % en poids, la quantité de co-tensioactif présent dans la formulation de remplissage étant de 0 à 60 % en poids, étant de 5 à 50 % en poids, ou étant de 10 à 40 % en poids, et la quantité d'huile présente dans la formulation de remplissage étant de 0 à 60 % en poids, ou étant de 5 à 50 % en poids, ou étant de 10 à 45 % en poids (où la quantité de formulation de remplissage comprenant la masse de l'ingrédient actif, du contre-ion lipophile, du véhicule lipidique et de tout autre excipient éventuel).

13. Composition selon l'une quelconque des revendications précédentes, le véhicule lipidique étant essentiellement constitué d'un tensioactif et d'un co-tensioactif éventuel, d'une formulation de remplissage constituée essentiellement d'un ingrédient actif (en équivalents de base libre) en une quantité de 2 à 40 % en poids, d'un contre-ion lipophile en une quantité allant de 2 à 40 % en poids, d'un tensioactif en une quantité allant de 10 à 96 % en poids, et d'un co-tensioactif en une quantité allant de 0 ou 0,1 à 60 % en poids.

14. Composition selon l'une quelconque des revendications précédentes comprenant un co-solvant.
